# EUROPEAN PATENT SPECIFICATION

(11) **EP 2 002 825 B1**
(45) Date of publication and mention of the grant of the patent: **22.05.2013**
(21) Application number: 07011695.9
(22) Date of filing: 14.06.2007
(51) Int. Cl.: A61K 9/14, A61K 31/365, A61K 9/48, A61K 9/16, A61K 31/337, C07D 305/12, A61P 3/04

(54) **Pharmaceutical compositions comprising orlistat**
Pharmazeutische Zubereitungen mit Orlistat
Compositions pharmaceutiques comportant de l'orlistat

(43) Date of publication of application: 17.12.2008
(73) Proprietor: KRKA, 8501 Novo Mesto (SI)
(72) Inventor: Zadnik, Jernej, 8350 Dolenjske Toplice (SI); Toporisic, Rebeka, 8250 Brezice (SI); Vehovec, Tanja, 4208 Sencur (SI); Smrkolj, Matej, 1411 Izlake Zagorje ob Savi (SI); Ocepek, Uros, 8000 Novo mesto (SI); Simonic, Igor, 8351 Straza pri Novom mestu (SI); Pelko, Mitja, 8000 Novo mesto (SI)
(74) Representative: Becker Kurig Straus

(56) References cited:
- EP-A- 1 803 714
- EP-A1- 0 638 317
- WO-A-00/13667
- WO-A-98/34607
- WO-A-03/047531
- WO-A-2005/026140
- WO-A-2007/021073
- CAIRA M R: "CRYSTALLINE POLYMORPHISM OF ORGANIC COMPOUNDS" TOPICS IN CURRENT CHEMISTRY, SPRINGER, BERLIN, DE, vol. 198, 1998, pages 163-208, XP001156954 ISSN: 0340-1022
- MORISSETTE SHERRY L ET AL: "HIGH-THROUGHPUT CRYSTALLIZATION: POLYMORPHS, SALTS, CO-CRYSTALS AND SOLCATES OF PHARMACEUTICAL SOLIDS" ADVANCED DRUG DELIVERY REVIEWS, AMSTERDAM, NL, vol. 56, no. 3, 2004, pages 275-300, XP009072233 ISSN: 0169-409X

## Description

The present invention pertains to pharmaceutical compositions comprising orlistat as an active ingredient. In particular, the present invention relates to such pharmaceutical compositions having an improved stability with respect to environmental conditions, such as increased temperature and moisture.

Orlistat as a lipstatin derivative, i.e. tetrahydrolipstatin, is a compound providing a pancreatic lipase-inhibiting activity and is used for the preparation of medicaments for preventing and treating obesity representing a condition affecting more than 30% of the adult population in the industrial world and represents a major public health problem, since the excessive accumulation of body fat may be dangerous and leads to different health problems. Examples of obesity linked diseases are type II diabetes, hypertension, hyperlipidemia, coronary heart disease, stroke, breast and colon cancer, sleep apnea, gallbladder disease, gastroesophageal reflux disease, fatty liver, gout, thromboembolism. Obesity is one of the main sectors of cardiovascular diseases. The levels of cholesterol, blood pressure, blood sugar and uric acid in obese people are usually higher than those of persons of normal weight. The morbidity from coronary heart disease among the overweight people is increased as well.

Chemical names of orlistat are (2S,3S,5S)-5-[(S)-2-formamido-4-methylvaleryloxy]-2-hexy-3-hydroxyhexadecanoic acid lactone or N-formyl-L-leucine-[2S-[2alpha(R*),3 beta]]-1-[(3-hexyl -4-oxo-2-oxetanyl)methyl]dodecyl ester. Orlistat exists in several crystalline forms. Analytic data and spectra characterizing the crystalline Form I and II of orlistat can be found in WO 2005/026140.

The synthesis of orlistat and its precursor lipstatin are already known in the art. Such complex secondary metabolites are advantageously produced by biochemical processes using suitable strains. An example of such a strain capable of producing lipstatin is Streptomyces toxytricini (Weibel E. et al., J. Antibiot. (1987), p. 1081). The chemical synthesis of orlistat is for example described by Hanessian, S. et al. (J. Org. Chem., 58 (1993), p. 7768-81). Other examples for suitable methods to manufacture lipstatin and orlistat are described for example in EP 0 129 748, EP 0 189 577, EP 1 028 115, WO 2004/003212, EP 0 803 576, WO 03/048335, EP 1 458 882, WO 2005/007639.

Conventional dosage forms of orlistat are inter alia disclosed in EP 0 129 748 and comprise for example, tablets or hard gelatine capsules.

EP 0 921 796 describes a composition comprising a plurality of pellets having a diameter in the range of from 0.25 to 2 mm wherein each particle comprises tetrahydrolipstatin, a stabilizer and at least one pharmaceutically acceptable excipient, wherein "stabilizer" refers to an agent having a rate of moisture uptake greater than the rate of moisture uptake for orlistat. Such stabilizer acts to retard hydrolytic degradation of the orlistat.

EP 1 307 264 refers to a composition comprising orlistat, a pharmaceutically acceptable bile acid sequestrant and pharmaceutically acceptable excipient.

EP 0 638 317 discloses pharmaceutical compositions for treating obesity, comprising inhibitors for glucosidase (i.e. acarbose) and/or amylase and an inhibitor for lipase, As such an inhibitor for lipase orlistat is mentioned.

WO 98/34607 discloses stable pharmaceutical compositions comprising tetrahydrolipstatin (also known as orlistat) in the form of pellets containing a stabilizer (e.g. polyvinylpyrrolidone) and pharmaceutically acceptable excipients such as microcrystalline cellulose.

WO 00/13667 refers to a combination product for reducing the side effects associated with lipostatin derivatives (e.g. orlistat). The product is in form of a tablet which comprises the lipstatin inhibitor (e.g. orlistat), a cellulose derivative, and diluents such as edible calcium salts.

Drawbacks of known compositions reside in formulation by powder mixing or conventional wet granulation procedures due to picking and sticking phenomena during tablet compression or encapsulation as well as induction of hydrolytic degradation due to pH. Due to the low melting point of about 44°C and structural properties, orlistat undergoes both hydrolytic and thermal degradation, particularly when stored in a humid atmosphere or above 35°C in a dry atmosphere (Helvetica Chemica Acta; 73 (1990; Pages 1022-1035)).

Accordingly, there is a need in orlistat containing products and dosage forms offering an increased stability towards moisture and heat during production and storage.

This problem has been solved by the provision of the present stable pharmaceutical formulation comprising orlistat mixed with a pharmaceutical excipient. The pharmaceutical excipient is characterized by a pH value in the range of from 4 to 8 when it is in the form of a 1% w/w aqueous solution or suspension at 25°C. Said pharmaceutical excipient is selected from the group consisting of calcium phosphate anhydrous, carboxymethylcellulose, ethyl cellulose and maize starch. The pharmaceutical formulation has further less than about 3.0 % of impurities (area %) at 30°C at a relative humidity of up to 65 % for a storage period of up to about 2 months.

It has been surprisingly found that the present composition may be prepared without the requirement of any other further agents, particularly stabilizers retarding hydrolytic degradation of orlistat. It should be, however, clear that other agents/compounds may be added in order to obtain a desired pharmaceutical form with suitable properties.

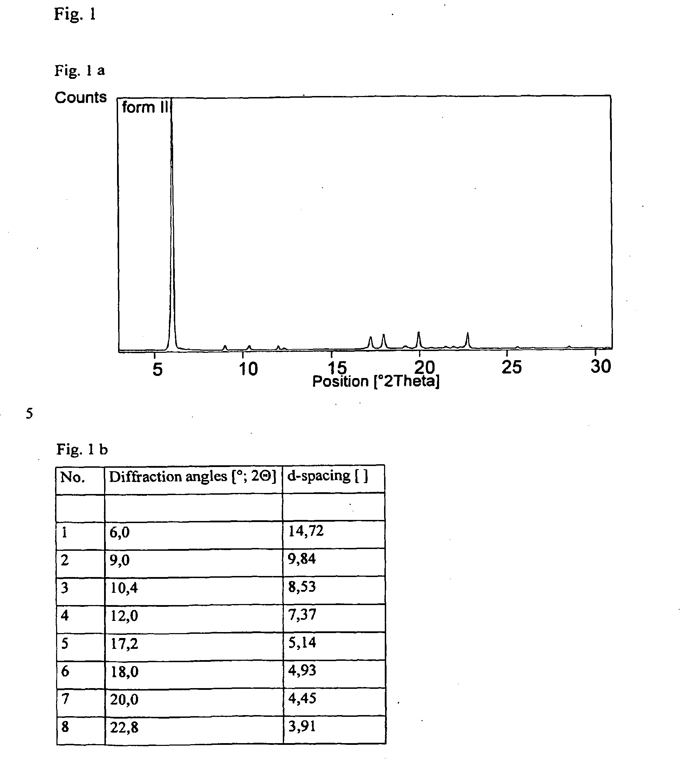

In the figures shows Fig. 1 the x-ray powder diffraction pattern of form III (Fig. 1 a) comprising the peaks (Fig. 1b). "d-spacing" is directed to the inter-planar spacing between successive atomic planes in the crystal; "Theta" (Θ) is the angle between the atomic plane and both the incident and reflected beams.

According to a preferred embodiment of the present invention a stable pharmaceutical formulation/composition comprising orlistat being mixed with a pharmaceutical excipient, wherein the pharmaceutical excipient is characterized by a pH value in the range of from 4 to 8 when it is in the form of a 1 % w/w aqueous solution or suspension at 25°C and which is selected from the group consisting of calcium phosphate anhydrous, carboxymethylcellulose, ethyl cellulose and maize starch, wherein the pharmaceutical formulation has less than about 3.0% (area %) of impurities at 30°C at a relative humidity of up to 65 % for a period of up to about 2 months.

For the preparation of the composition pure orlistat may be used as a starting material, i.e. Orlistat having purity within the range of 99 to 100 %, preferably higher than 99.5 % and most preferably higher than 99.8 %. Orlistat may be prepared according to any desired method, for example according to a method described in EP 0 129 748, EP 0 189 577, EP 1 028 115, WO 2004/003212, EP 0 803 576, WO 03/048335, EP 1 458 882, WO 2005/007639 or by means of a catalytic hydrogenation of lipstatin as described in EP 05 028 489. Alternative processes for preparing polymorphic forms I and II are for example disclosed in WO 2005/026140. The different polymorphic forms of orlistat may be in general obtained by crystallization from different solvents, different cooling and/or aging conditions.

Orlistat may have an average particle size in the range of 5 to 750 µm, preferably 10 to 500 µm, more preferably 50 to 400 µm. The term "average particle size" or "particle size" as used herein refers to the volume mean diameter of particles, which may be determined by suitable means known to the skilled person such as a Malvern Mastersizer.

The pharmaceutical excipient may be selected according to any equilibrium moisture content from 0.1 % to 20 % at 25 °C at relative humidity of 50 %. The pharmaceutical excipient of pH value 4 to 6 may be selected for an equilibrium moisture content of less than 11 % and pharmaceutical excipient of pH value 7 to 8 may be selected for an equilibrium moisture content of less than 0.2 %.

The term impurity refers hereby to hydrolytic orlistat degradation products, such as (2S,3R,SS)-5-[(S)-2-formylamino-4-methyl-pentanoyloxy]-hexyl-3-hydroxyhexadecanoic acid, (2S,3S,5S)-5-[(N-formyl-L-leucyl)oxy]-2-hexyl-3-hydroxyhexadecanoic acid, N-formyl-L-leucine (3S,4R,6S)-tetrahydro-3-hexyl-2-oxo-6-undecyl-2H-pyran-4-yl ester, (S)-3-hexyl-5,6-dihydro-6-undecyl-2H-pyran-2-one and N-formyl-L-leucine (S,E)-1-undecyl-3-decenyl ester. The amount of other orlistat degradation products is negligible and does not exceed about 0.2 % (area %). It will be appreciated that the accuracy for the determination of impurities will depend on the analysis method employed.

The present pharmaceutical compositions/formulations are in solid dosage form. Exemplary solid dosage forms include tablets, capsules, sachets, lozenges, powders, pills, pellets, or granules. The solid dosage form may be, for example, a immediate release dosage form, a fast melt dosage form, orally disintegrating dosage form, modified release dosage form, lyophilized dosage form, delayed release dosage form, extended release dosage form, prolonged release dosage form, pulsatile dosage form, mixed immediate and modified release dosage form, or a combination thereof. Solid dosage forms are preferred. More preferably, the solid dosage form is an immediate release dosage form offering advantages regarding the bioavailability of the active compound. The term bioavailability describes the rate and extent to which the active ingredient or active moiety is absorbed from a drug product and becomes available at the site of action. The bioavailability of orally ingested drugs is determined by factors, which include the nature of the molecule, its stability, and the formulation administered - and in the patient - such as a reduced intestinal surface area as a result of colic disease or intestinal resection and whether or not the drug is taken with a meal. Factors influencing the bioavailability may include, but are not limited to a poor absorption from the gastrointestinal tract, hepatic first-pass effect and degradation of the drug prior to reaching system circulation.

In case an immediate release dosage form is chosen, it will be clear for the skilled person that the amount of release controlling agent(s) to be used in forming the outer portion will be determined based on various parameters such as the desired delivery properties, including the amount of active ingredient or substance to be delivered, the a active ingredient or substance release rate desired, and the size of the micro matrix particles.

The dosage form may be manufactured according to the following procedure: The core particles may be produced in accordance with usual techniques in which the active ingredient or substance and one or more release controlling agents are mixed and granulated by adding solvent in a low or high shear mixer or by fluidized bed granulator. The granulate is dried, for example in a fluidized bed dryer. The dried granulate is sized. The sizing of the micromatrix particles may be performed by using an oscillating granulator, comminuting mill or any other conventional method. The sieve used for the sizing may have openings from 0.25 mm to 5 mm. Alternatively the core particles can be made by extrusion, spheronization, melt granulation or by roller compaction. The core particles may be coated by a solution of one or more release controlling agents by any known method, including spray application. Spraying can be carried out using a fluidized bed coater (preferably Wurster coating), or in a pan coating system. Alternatively the coating of the core particles with one or more rate controlling agents can be done by hot melt process using a granulator or fluidized bed coater (preferably Wurster coating), or in a pan coating system. The compression of micro tablets is carried out on usual compression machines (e. g. machines by Manesty, Cadmach or Kilian). The micro tablets can be made of various sizes and shapes like round, oval, oblong, capsule shaped, triangular, square, etc. The preferred shape of the micro tablet is round, biconvex and the preferred diameter of the micro tablet is 1.5 mm to 9.5 mm.

Pharmaceutical dosage forms comprising orlistat can be prepared by a process comprising the steps of mixing orlistat according to the present invention with at least one pharmaceutically acceptable excipient and forming the mixture into a pharmaceutical dosage form. Orlistat and the one or more excipients may be mixed in the presence or in the absence of solvent.

The manufacturing process for oral solid capsules can be a conventional process comprising the steps of preparing particles, drying, final blending and/or capsule filling. These particles are typically in the form of granules or pellets.

The active ingredient may be mixed with excipients for preparing granules and then be subjected to granulation, or alternatively granules can be formed without the active ingredient and these granules and optional further ingredients are then mixed with the active ingredient in the final blending step to form e.g. a capsule filling mixture. Granules can be produced by common granulation techniques, such as wet granulation or dry granulation methods.

Suitable excipients for preparing granules are preferably selected from the following excipients diluents, binding agents, disintegrants, lubricants, sweeteners, glidants, flavourings, colouring agents, depending on the dosage form desired.

The granulation can be performed either by using solvents (wet granulation) or by dry granulation. Any organic solvent may be used in the wet granulation step using state of the art granulation equipment such as fluid bed granulators, high or low shear mixers. The obtained granulate can be further dried by using microwave, fluid bed or convection dryers, drying chambers with or without using reduced pressure or vacuum. Dry granulation can be performed by using compactors or briquetting techniques. Preferred solvents for wet granulation are selected from water and/or alcohols.

Preferably a wet granulation process is used, in which the active compound in powder form is presented in a suitable granulator and subsequently moistened or sprayed with molten material. The shear forces applied lead to an intensive mixing of the powder and, with the addition of binder solutions, to the rapid formation of high-density granulates. Granulation is required to improve the flow of powder mixtures and mechanical properties of tablets. Granules are usually obtained by adding liquids (binder or solvent solutions). Larger quantities of granulating liquid produce a narrower particle size range and coarser and harder granules, i.e. the proportion of fine granulate particles decreases. The optimal quantity of liquid needed to get a given particle size should be known in order to keep a batch-to-batch variations to a minimum. Wet granulation improves flow, reduced stickiness, compressibility, bioavailability, homogeneity, electrostatic properties, and stability of solid dosage forms.

The granules obtained according to above described methods can be encapsulated into hard gelatine capsules or capsules consisting of hydroxypropylmethyl cellulose.

As an alternative to the encapsulation of granules, capsules can be filled with the dry mixture, wherein dry mixture is prepared using conventional manufacturing processes comprising mixing of active ingredient and above excipients, followed by final blending and/or capsule filling.

Formation of hydrolytic impurities may be further diminished by either packaging the pharmaceutical composition under reduced pressure, packaging in an inert gas atmosphere, using a coating affording protection and stability of the pharmaceutical composition from environmental influences or by using a packaging wherein the contact between the pharmaceutical composition and humidity is reduced by the means of substances capable to adsorb water. This may lead to an additional stabilisation of the active compound, since hydrolysis mediated degradation/decomposition reactions of orlistat are further reduced

Reduced pressure may be obtained e.g. by creating a partial vacuum by means of a suitable pump or by partial freezing or liquefying the atmosphere, by the use of an inert gas atmosphere, wherein as an inert gas nitrogen or argon may serve for example, or by the use of absorbents. Absorbents may be selected from the group of commercially available absorbents such as humidity-activated oxygen absorbers, ultraviolet-radiation-activated absorbers, radiation-activated absorbers, microwaves-radiation-activated absorbers, absorbers activated by a combination of activation processes or absorbers without necessity of activation. The formulation may be enclosed in a substantially gas exchange non-permeable material, which is preferably selected from the group consisting of an Al/Al blister, an Al-polychloro-3-fluoroethylene homopolymer/PVC laminate blister or a bottle.

The contact between the pharmaceutical composition and humidity containing environment may be reduced by the use of coatings. Film coatings, which prevent environmental gases and humidity to ingress into the cores may be used.

The present orlistat compositions have been proven to be stable at temperatures <30°C at a relative humidity of up to 65 % in an open container for a period of up to about 2 months.

According to an embodiment the impurities are selected from (2S,3R,5S)-5-[(S)-2-formylamino-4-methyl-pentanoyloxy]-hexyl-3-hydroxyhexadecanoic acid, (2S,3S,5S)-5-[(N-formyl-L-leucyl)oxy]-2-hexyl-3-hydroxyhexadecanoic acid and N-formyl-L-leucine (3S,4R,6S)-tetrahydro-3-hexyl-2-oxo-6-undecyl-2H-pyran-4-yl ester. The pharmaceutical composition has impurities less than about 3.0 %.

According to another embodiment (2S,3R,5S)-5-[(S)-2-formylamino-4-methyl-pentanoyloxy]-hexyl-3-hydroxyhexadecanoic acid is contained in amounts of not more than 1.5%.

According to still another embodiment (2S,3S,5S)-5-[(N-formyl-L-leucyl)oxy]-2-hexyl-3-hydroxyhexadecanoic acid is contained in amounts of not more than 0.3%.

According to another embodiment N-formyl-L-leucine (3S,4R,6S)-tetrahydro-3-hexyl-2-oxo-6-undecyl-2H-pyran-4-yl ester is contained in amounts of not more than 1.0 %.

According to still another embodiment the ratio of orlistat and pharmaceutical excipient is in the range of from 0.01 : 1 to 100 : 1. Preferably, the ratio of orlistat and pharmaceutical excipient

is in the range of from 0.1 : 1 to 10 : 1, more preferably the ratio is in the range of from 0.4 : 1 and 6 : 1.

According to yet another embodiment the pharmaceutical excipient has an equilibrium moisture content bellow 11 % at 25°C at relative humidity of 50 %.

Also disclosed is a pharmaceutical composition comprising orlistat polymorphic form III. Orlistat polymorphic form III is may be essentially free from other polymorphic or amorphous forms of orlistat. Essentially free from other polymorphic forms or amorphous forms designates an amount in the range of from <10% to 0.1% by weight, which has been found to be not critical for the stability of the composition. The amount of said other forms may be in the range of from <5% to 0.5% by weight and more preferably in the range of from <2% to 0.5% by weight.

Polymorphic form III of orlistat has been found particularly suitable for inclusion in pharmaceutical compositions. The same issue applies for the known polymorphic forms I and II of orlistat in case the pharmaceutical composition is prepared by providing polymorphic forms I or II and a first amount of at least one excipient, granulating (optionally in the presence of an organic solvent); and drying according to the present process. The pharmaceutical excipient being selected from the group consisting of calcium phosphate anhydrous, carboxymethylcellulose, ethyl cellulose and maize starch is characterized by a pH value in the range of from 4 to 8 when it is in the form of a 1 % aqueous solution or suspension, wherein the pharmaceutical formulation has less than about 3.0% of impurities at 30°C at a relative humidity of up to 65 % for a period of up to about 2 months.

It is also possible to use the mixture of polymorph I, II and III, such for example III and I, III and II, and I and II.

It has been unexpectedly found that by use of polymorphic form III of orlistat for the preparation of a pharmaceutical composition the overall impurities contained are less or equal to 3.0% by weight. The present process bestows another possibility to reduce the amount of overall impurities contained to the same value as indicated above in case polymorphic forms I or II are used.

Without wishing to be bound by any theory it is presently assumed that polymorphic form III of orlistat is distinguished from other known polymorphic forms by a more complex crystal structure rendering the compound less accessible to water reducing thereby rearrangement reactions and the influence of higher temperature residing in an increased of such reactions.

According to another preferred embodiment of the present invention a process for the preparation of the present pharmaceutical composition is provided, comprising the steps of providing polymorphic form I or II of orlistat and a first amount of one excipient having a pH value in the range of from 4 to 8 when it is in the form of a 1% w/w aqueous solution or suspension at 25°C; granulating (optionally in the presence of an organic solvent); optionally adding a second amount of the above excipient(s) and drying.

The organic solvent used may be any suitable volatile organic solvent used in the preparation of pharmaceutical compositions. Preferably, said organic solvent is ethanol.

According to still another embodiment a pharmaceutical composition/formulation is provided, which is obtained by the present process.

The average particle size is determined with a Malvern Mastersizer. The particles to be subjected to the particle size measurement are first suspended in isoparaffin oil (e.g. Isopar^{®}) and then subjected to size determination in a Malvern Mastersizer MS 2000 instrument. Usually, 100-800 mg of substance are dispersed in 5-8 ml of oil. According to manufacturer's information, the Malvern Mastersizers allows measurement of particle size distributions within the range of 20 nm to 2000 µm, with a precision of better than 0.5%. Particle sizes are determined by measuring the angular distribution of laser light scattered by a homogeneous suspension of particles. The size distribution is determined from the light scattering data using the theory of light scattering developed by Gustav Mie.

### Example 1 - Formulation containing orlistat prepared by granulation

| Ingredient | mg/capsule |
|---|---|
| Orlistat | 120 |
| Calcium Phosphate, Dibasic Anhydrous | 150 |
| Total | 270 |

1. Orlistat and Calcium Phosphate, Dibasic Anhydrous (pH of 1 % w/w aqueous suspension of calcium phosphate was 7.34) are sieved, mixed in high share mixer (225.6 g of mixture) and granulated with the ethanol as granulating liquid.
2. Granules are dried at or below 25°C (input air 28-30 °C) and passed through 18 Mesh screen. The loss on drying (measurement performed by Mettler Tolledo HR73 halogen moisture analyzer at 85 °C for 20 minutes) of the obtained granules was 0.78 % by weight
3. Additional Calcium Phosphate, Dibasic Anhydrous (of the same type) is added (44.4 g) to the granulate and mixed. The mixture was dried additionally. The loss on drying 0.05 % by weight was obtained.
4. Mixture was filled in a hydroxypropyl methyl cellulose based capsule (No. 1).

One part of the capsules was packed into Alu-Alu blister packs and the other part of the capsules was not packed.

In stress stability testing the amount of hydrolytic impurities has not changed (analyzed by HPLC) after 5 and 9 weeks for closed containers (Alu-Alu blister packs) at 30°C/65 % RH nor in opened container after 4 and 8 weeks at the same conditions.

| Impurity | t0 | 30°C/65%RH - AluAlu blister | | 30°C/65%RH - opened container | |
|---|---|---|---|---|---|
| | | 5 weeks | 9 weeks | 4 weeks | 8 weeks |
| H1 | bld | bld | bld | bld | bld |
| H2 | bld | bld | bld | bld | bld |
| H3 | bld | blq | blq | blq | bld |
| Total | 1.09* | 1.07* | 1.09* | 1.07* | 1.09* |

| | | | | | |
|---|---|---|---|---|---|
| bld: bellow the limit of detection (< 0.05%) blq: bellow the limit of quantification (≤ 0.10%) *calculated on related substances ≥ 0.1% | | | | | |

### Example 2 - Formulation containing orlistat prepared by granulation

| Ingredient | mg/capsule |
|---|---|
| Orlistat | 120 |
| Carboxymethylcellulose Sodium | 150 |
| Total | 270 |

1. Orlistat and Carboxymethylcellulose Sodium (pH of 1 % w/w aqueous solution of carboxymethylcellulose sodium was 6.31) are sieved, mixed in high share mixer (225.6 g of mixture) and granulated with the ethanol as granulating liquid.
2. Granules are dried at or below 25°C (input air 28-30 °C) and passed through 18 Mesh screen. The loss on drying (measurement performed by Mettler Tolledo HR73 halogen moisture analyzer at 85 °C for 20 minutes) of the obtained granules was 1.17 % by weight
3. Additional Carboxymethylcellulose Sodium (of the same type) is added (44.4 g) to the granulate and mixed. The mixture was dried additionally. The loss on drying 0.17 % by weight was obtained.
4. Mixture was filled in a hydroxypropyl methyl cellulose based capsule (No. 1)

One part of the capsules was packed into Alu-Alu blister packs and the other part of the capsules was not packed.

In stress stability testing the amount of hydrolytic impurities has not changed (analyzed by HPLC) after 5 and 9 weeks for closed containers (Alu-Alu blister packs) at 30°C/65 % RH nor in opened container after 4 and 8 weeks at the same conditions.

| Impurity | t0 | 30°C/65%RH - AluAlu blister | | 30°C/65%RH-opened container | |
|---|---|---|---|---|---|
| | | 5 weeks | 9 weeks | 4 weeks | 8 weeks |
| H1 | bld | bld | bld | bld | bld |
| H2 | bld | bld | bld | bld | bld |
| H3 | bld | bld | bld | bld | bld |
| Total | 1.06* | 1.01* | 0.87* | 1.02* | 1.00* |

| | | | | | |
|---|---|---|---|---|---|
| bld: bellow the limit of detection ( ≤ 0.05%) blq: bellow the limit of quantification (≤ 0.10%) *calculated on related substances ≥ 0.1% | | | | | |

### Example 3 - Formulation containing orlistat prepared by granulation

| Ingredient | mg/capsule |
|---|---|
| Orlistat | 120 |
| Ethyl cellulose 20 cp | 150 |
| Total | 270 |

1. Orlistat and Ethyl cellulose (pH of 1 % w/w aqueous suspension of ethyl cellulose was 6.71) are sieved, mixed in high share mixer (225.6 g of mixture) and granulated with the ethanol as granulating liquid.
2. Granules are dried at or below 25°C (input air 28-30 °C) and passed through 18 Mesh screen. The loss on drying (measurement performed by Mettler Tolledo HR73 halogen moisture analyzer at 85 °C for 20 minutes) of the obtained granules was 0.62 % by weight
3. Additional Ethyl cellulose (of the same type) is added (44.4 g) to the granulate and mixed. The mixture was dried additionally. The loss on drying 0.23 % by weight was obtained.
4. Mixture was filled in a hydroxypropyl methyl cellulose based capsule (No. 1)

One part of the capsules was packed into Alu-Alu blister packs and the other part of the capsules was not packed.

In stress stability testing the amount of hydrolytic impurities has not changed (analyzed by HPLC) after 5 and 9 weeks for closed containers (Alu-Alu blister packs) at 30°C/65 % RH nor in opened container after 4 and 8 weeks at the same conditions.

| impurity | t0 | 30°C/65%RH- AluAlu blister | | 30°C/65%RH - opened container | |
|---|---|---|---|---|---|
| | | 5 weeks | 9 weeks | 4 weeks | 8 weeks |
| H1 | bld | bld | bld | bld | bld |
| H2 | bld | bld | bld | bld | bld |
| H3 | bld | bld | bld | bld | bld |
| Total | 1.07* | 1.03* | 1.06* | 1.02* | 1.03* |

| | | | | | |
|---|---|---|---|---|---|
| bld: bellow the limit of detection (≤ 0.05%) blq: bellow the limit of quantification (≤ 0.10%) *calculated on related substances ≥ 0.1% | | | | | |

### Example 4 - Formulation containing orlistat prepared by granulation

| Ingredient | mg/capsule |
|---|---|
| Orlistat | 120 |
| Maize starch, dried | 150 |
| Total | 270 |

1. Orlistat and Maize starch (pH of 1 % w/w aqueous suspension of maize starch was 4.53), dried are sieved, mixed in high share mixer (225.6 g of mixture) and granulated with the ethanol as granulating liquid.
2. Granules are dried at or below 25°C (input air 28-30 °C) and passed through 18 Mesh screen.
3. Additional Maize starch, dried (of the same type) is added (44.4 g) to the granulate and mixed. The mixture was dried additionally. The loss on drying 0.07 % by weight was obtained.
4. Mixture was filled in a hydroxypropyl methyl cellulose based capsule (No. 1)

One part of the capsules was packed into Alu-Alu blister packs and the other part of the capsules was not packed.

In stress stability testing the amount of hydrolytic impurities has not changed (analyzed by HPLC) after 5 and 9 weeks for closed containers (Alu-Alu blister packs) at 30°C/65 % RH nor in opened container after 4 and 8 weeks at the same conditions.

| Impurity | t0 | 30°C/65%RH- AluAlu blister | | 30°C/65%RH - opened container | |
|---|---|---|---|---|---|
| | | 5 weeks | 9 weeks | 4 weeks | 8 weeks |
| H1 | bld | bld | bld | bld | bld |
| H2 | bld | bld | bld | bid | bld |
| H3 | bld | blq | blq | blq | blq |
| Total | 1.10* | 1.08* | 1.09* | 1.11* | 1.14* |

| | | | | | |
|---|---|---|---|---|---|
| bld: bellow the limit of detection (≤ 0.05%) blq: bellow the limit of quantification (≤ 0.10%) *calculated on related substances ≥ 0.1% | | | | | |

### Example 5 - Formulation comprising orlistat of polymorphic form III (Reference Example)

| Ingredient | mg/capsule |
|---|---|
| orlistat | 120 |
| PEG 4000 | 120 |
| Total | 240 |

Formulation was prepared by supercritical fluid technology using the method of PGSS (particles from gas saturated solutions) with the use of CO₂. Physical mixture of orlistat and PEG 4000 was put into the high pressure vessel. Temperature and pressure of the CO₂ in the vessel were raised to 40°C and 160 bar. Mixture was then mixed for 1 hour. Melt with dissolved CO₂ was then sprayed through the nozzle to normal pressure. Particles of average size of 60 µm were prepared comprising more than 90 % of orlistat of form III as shown in Figure 1.

## Claims

1. A stable pharmaceutical formulation comprising orlistat mixed with a single pharmaceutical excipient, which excipient has a pH value in the range of from 4 to 8 when it is in the form of a 1 % w/w aqueous solution or suspension at 25°C and which excipient is selected from the group consisting of calcium phosphate anhydrous, carboxymethylcellulose, ethyl cellulose and maize starch, wherein the pharmaceutical formulation has less than about 3.0 % of impurities (area %) at 30°C at a relative humidity of up to 65 % for a period of up to about 2 months.

2. Pharmaceutical composition according to claim 1, wherein the impurities are selected from (2S,3R,5S)-S-[(S)-2-formylamino-4-methyl-pentanoyloxy]-hexyl-3-hydroxyhexadecanoic acid, (2S,3S,5S)-S-[(N-formyl-L-leucyl)oxy]-2-hexyl-3-hydroxyhexadecanoic acid and N-formyl-L-leucine (3S,4R,6S)-tetrahydro-3-hexyl-2-oxo-6-undecyl-2H-pyran-4-yl ester.

3. Pharmaceutical composition according to any of the preceding claims, wherein the content of (2S,3R,5S)-5-[(S)-2-formylamino-4-methyl-pentanoyloxy]-hexyl-3-hydroxyhexadecanoic acid is not more than 1.5 %.

4. Pharmaceutical composition according to claim 1 or 2, wherein the content of (2S,3S,5S)-5-[(N-formyl-L-leucyl)oxy]-2-hexyl-3-hydroxyhexadecanoic acid is not more than 0.3 %.

5. Pharmaceutical composition according to claim 1 or 2, wherein the content of N-formyl-L-leucine (3S,4R,6S)-tetrahydro-3-hexyl-2-oxo-6-undecyl-2H-pyran-4-yl ester is not more than 1.0 %.

6. Pharmaceutical composition according to any of the preceding claims, wherein the ratio of orlistat and pharmaceutical excipient is in the range of from 0.01 : 1 to 100 : 1.

7. Pharmaceutical composition according to claim 6, wherein the ratio of orlistat and pharmaceutical excipient is in the range of from 0.1 : 1 to 10 : 1.

8. Pharmaceutical composition according to claim 6, wherein the ratio of orlistat and pharmaceutical excipient is in the range of from 0.4 : 1 and 6 : 1.

9. Pharmaceutical composition according to any of the preceding claims, wherein the pharmaceutical excipient has an equilibrium moisture content below 11 % at 25°C at relative humidity of 50 %.

10. Process for the preparation of a pharmaceutical composition according to claim 1, comprising the steps of:
a.) providing polymorphic form I or II of orlistat and a first amount of one excipient having a pH value in the range of from 4 to 8 when it is in the form of a 1 % w/w aqueous solution or suspension at 25°C;
b.) granulating, optionally in the presence of an organic solvent;
c.) optionally adding a second amount of the excipient defined in a.); and
d.) drying.

11. The process according to claim 10, wherein said organic solvent is ethanol.

12. Pharmaceutical composition obtained by the process according to claim 10 or 11.

## Patentansprüche

1. Stabile pharmazeutische Formulierung umfassend mit einem einzelnen pharmazeutischen Hilfsstoff vermischtes Orlistat, worin der Hilfsstoff einen pH Wert in dem Bereich von 4 bis 8 aufweist, wenn er in der Form einer 1 Gew.-% wässrigen Lösung oder Suspension bei 25°C vorliegt, und worin der Hilfsstoff ausgewählt ist unter der Gruppe bestehend aus wasserfreiem Calciumphosphat, Carboxymethylcellulose, Ethylcellulose und Maisstärke, worin die pharmazeutische Formulierung weniger als ungefähr 3,0 % Verunreinigungen (Flächen %) bei 30°C bei einer relativen Feuchtigkeit von bis zu 65 % für einen Zeitraum von bis zu ungefähr 2 Monaten aufweist.

2. Pharmazeutische Zusammensetzung nach Anspruch 1, worin die Verunreinigungen ausgewählt sind unter (2S,3R,5S)-5-[(S)-2-Formylamino-4-methyl-pentanoyloxy]-hexyl-3-hydroxyhexadecansäure, (2S,3S,5S)-5-[(N-Formyl-L-leucyl)oxy]-2-hexyl-3-hydroxyhexadecansäure und N-Formyl-L-Leucin (3S,4R,6S)-Tetrahydro-3-hexyl-2-oxo-6-undecyl-2H-pyran-4-yl-ester.

3. Pharmazeutische Zusammensetzung nach einem der vorstehenden Ansprüche, worin der Gehalt an (2S,3R,5S)-5-[(S)-2-Formylamino-4-methyl-pentanoyloxy]-hexyl-3-hydroxyhexadecansäure nicht mehr als 1,5 % beträgt.

4. Pharmazeutische Zusammensetzung nach Anspruch 1 oder 2, worin der Gehalt an (2S,3S,5S)-5-[(N-Formyl-L-leucyl)oxy]-2-hexyl-3-hydroxyhexadecansäure nicht mehr als 0,3 % beträgt.

5. Pharmazeutische Zusammensetzung nach Anspruch 1 oder 2, worin der Gehalt an N-Formyl-L-Leucin (3S,4R,6S)-Tetrahydro-3-hexyl-2-oxo-6-undecyl-2H-pyran-4-yl-ester nicht mehr als 1,0 % beträgt.

6. Pharmazeutische Zusammensetzung nach einem der vorstehenden Ansprüche, worin das Verhältnis von Orlistat zu pharmazeutischem Hilfsstoff in dem Bereich von 0,01 : 1 bis 100 : 1 liegt.

7. Pharmazeutische Zusammensetzung nach Anspruch 6, worin das Verhältnis von Orlistat zu pharmazeutischem Hilfsstoff in dem Bereich von 0,1 : 1 bis 10 : 1 liegt.

8. Pharmazeutische Zusammensetzung nach Anspruch 6, worin das Verhältnis von Orlistat zu pharmazeutischem Hilfsstoff in dem Bereich von 0,4 : 1 und 6 : 1 liegt.

9. Pharmazeutische Zusammensetzung nach einem der vorstehenden Ansprüche, worin der pharmazeutische Hilfsstoff einen Gleichgewichtsfeuchtigkeitsgehalt von unter 11 % bei 25°C bei einer relativen Feuchtigkeit von 50 % aufweist.

10. Verfahren zur Herstellung einer pharmazeutischen Zusammensetzung nach Anspruch 1, umfassend die Schritte:
a.) Bereitstellen von Orlistat in polymorpher Form I oder II und eine erste Menge eines Hilfsstoffs mit einem pH Wert in dem Bereich von 4 bis 8, wenn er in der Form einer 1 Gew.-% wässrigen Lösung oder Suspension bei 25°C vorliegt;
b.) Granulieren, wahlweise in der Anwesenheit eines organischen Lösungsmittels;
c.) wahlweise Hinzufügen einer zweiten Menge des in a.) festgelegten Hilfsstoffs; und
d.) Trocknen.

11. Verfahren nach Anspruch 10, worin das organische Lösungsmittel Ethanol ist.

12. Pharmazeutische Zusammensetzung, die durch das Verfahren nach Anspruch 10 oder 11 erhalten wird.

## Revendications

1. Une formulation pharmaceutique stable comprenant de l'orlistat en mélange avec un excipient pharmaceutique unique, lequel excipient a une valeur de pH dans la plage de 4 à 8 lorsqu'il est sous forme d'une solution ou suspension aqueuse à 1% en poids à 25° C et lequel excipient est choisi à partir du groupe consistant en phosphate de calcium anhydre, carboxyméthylcellulose, éthylcellulose et amidon de maïs, dans laquelle la formulation pharmaceutique comporte moins d'environ 3.0% d'impuretés (% en surface) à 65% d'humidité relative et à 30° C, sur une période allant jusqu'à environ 2 mois.

2. Composition pharmaceutique selon la revendication 1, dans laquelle les impuretés sont choisies à partir de l'acide (2S, 3R, 5S) -5 - [(S)-2-formylamino-4-méthyl-pentanoyloxy]-hexyl-3-hydroxyhexa-décanoïque, l'acide (2S, 3S, 5S) -5 - [(N-formyl-L-leucyl)-oxy]-2-hexyl-3-hydroxyhexadécanoïque et l'ester de N-formyl-L-leucine (3S, 4R, 6S)-tétrahydro-3-hexyl -2-oxo-6-undécyl-2H-pyranne-4-yle.

3. Composition pharmaceutique selon l'une des revendications précédentes, dans laquelle la teneur en acide (2S, 3R, 5S) -5 - [(S)-2-formylamino-4-méthyl-pentanoyloxy]-hexyl-3-hydroxyhexa-décanoïque n'est de pas plus de 1.5%.

4. Composition pharmaceutique selon la revendication 1 ou 2, dans lequel la teneur en acide (2S, 3S, 5S) -5 - [(N-formyl-L-leucyl)-oxy]-2-hexyl-3-hydroxyhexadécanoïque l'acide n'est de pas plus de 0.3%.

5. Composition pharmaceutique selon la revendication 1 ou 2, dans lequel la teneur en ester de N-formyl-L-leucine (3S, 4R, 6S)-tétrahydro-3-hexyl-2-oxo-6-undécyl-2H-pyranne-4-yle ester n'est de pas plus de à 1.0%.

6. Composition pharmaceutique selon l'une des revendications précédentes, dans laquelle le rapport de l'orlistat à l'excipient pharmaceutique est dans la plage de 0.01: 1 à 100: 1.

7. Composition pharmaceutique selon la revendication 6, dans laquelle le rapport de l'orlistat à l'excipient pharmaceutique est dans la plage de 0.1: 1 à 10: 1.

8. Composition pharmaceutique selon la revendication 6, dans laquelle le rapport de l'orlistat à l'excipient pharmaceutique est dans la plage de 0.4: 1 à 6:1.

9. Composition pharmaceutique selon l'une quelconque des revendications précédentes, dans laquelle l'excipient pharmaceutique a une teneur en humidité à l'équilibre en dessous de 11% à 25° C pour une humidité relative de 50%.

10. Procédé pour la préparation d'une composition pharmaceutique selon la revendication 1, comprenant les étapes de :
a) fournir la forme polymorphe 1 ou II d'orlistat et une première quantité d'un excipient ayant une valeur de pH dans la plage de 4 à 8 lorsqu'il est sous forme d'une solution ou suspension aqueuse à 1% en poids à 25° C;
b) effectuer la granulation, le cas échéant en présence d'un solvant organique;
c) ajouter le cas échéant une seconde quantité de l'excipient défini sous a); et
d) sécher.

11. Le procédé selon la revendication 10, dans lequel ledit solvant organique est l'éthanol.

12. Composition pharmaceutique obtenue par le procédé selon la revendication 10 ou 11.
